Europäisches Patentamt

European Patent Office

Office européen des brevets

(19)

(11) Veröffentlichungsnummer: **0 307 002 B1**

(12) **EUROPÄISCHE PATENTSCHRIFT**

(45) Veröffentlichungstag der Patentschrift: 21.11.91

(21) Anmeldenummer: 88115353.0

(22) Anmeldetag: 10.05.84

(51) Int. Cl.⁵: **A61K 35/16, C07K 3/20**

(60) Veröffentlichungsnummer der früheren Anmeldung nach Art. 76 EPÜ: **0 129 534**

(54) Verfahren zur Herstellung eines Antithrombin III-Konzentrates.

(30) Priorität: 20.05.83 AT 1859/83

(43) Veröffentlichungstag der Anmeldung:
15.03.89 Patentblatt 89/11

(45) Bekanntmachung des Hinweises auf die
Patenterteilung:
21.11.91 Patentblatt 91/47

(84) Benannte Vertragsstaaten:
AT BE CH DE FR GB IT LI NL SE

(56) Entgegenhaltungen:

CHEMICAL ABSTRACTS, Band 97, Nr. 1, 5.
Juli 1982, Zusammenfassung Nr. 319p, Seite
32, Columbus, Ohio, US; Y. OKAJIMA et al.:
"Studies on the effect of protamine on antithrombin II-heparin complex", & KETSUEKI
TO MYAKKAN 1981, 12(3), 407-11

DIE MAKROMOL. CHEM., Band 183, 1982, Seiten 2305-2310; K. ZEITZ et al.:
"Immobilisierung von Salmin an CNBr-
Sepharose 4B"

(73) Patentinhaber: IMMUNO Aktiengesellschaft
Industriestrasse 67
A-1221 Wien(AT)

(72) Erfinder: Eibl, Johann, Dr.
Gustav Tschermakgasse 2
A-1180 Wien(AT)
Erfinder: Hetzl, Ernst, Dr. Dipl.-Ing.
Bergheidengasse 8/1/9
A-1130 Wien(AT)
Erfinder: Linnau, Yendra, Dr.
Lavendlweg 24
A-1220 Wien(AT)

(74) Vertreter: Wolfram, Gustav, Dipl.-Ing.
Schwindgasse 7 P.O. Box 205
A-1041 Wien(AT)

## Beschreibung

Die Erfindung betrifft ein Verfahren zur Herstellung eines Antithrombin III-Konzentrates, wobei zuerst Humanplasma oder Antithrombin III-haltige Plasmafraktionen unter Bildung eines Antithrombin III-Heparin- bzw. Antithrombin III-Heparinoid-Komplexes mit Heparin bzw. Heparinoid versetzt wird und anschließender Spaltung des Antithrombin III-Heparin-bzw. Antithrombin III-Heparinoid-Komplexes mit Protamin.

Es ist bekannt, daß Antithrombin III pharmazeutisch wertvolle Eigenschaften aufweist; insbesondere wird durch die Gegenwart von Antithrombin III bei der Herstellung und Lagerung von Konzentraten hochwirksamer Gerinnungsfaktoren und anderer Plasmaproteine mit biologischer Wirksamkeit enzymatisch bedingte Proteinveränderungen, die unerwünschte Nebenreaktionen am Patienten zur Folge haben können, unterdrückt. Weitere pharmazeutisch wertvolle Eigenschaften von Antithrombin III sind die Wirksamkeit gegenüber Thromboembolismus bei angeborenem Mangel an Antithrombin III sowie bei Risikopatienten, bei denen unter Heparintherapie Antithrombin III-Abfall eintritt.

In der US-A-4,087,415 sowie in R.D. Rosenberg et al., J. Biol. Chem. 248, 6490 (1973) werden Verfahren zur Gewinnung von Antithrombin III beschrieben, die eine Adsorption an Al(OH)$_3$, Elution mit Phosphat/EDTA, weitere Reinigung durch Fraktionierung mit Pluronic oder Polyäthylenglykol bzw. durch Gelfiltration, Ionenaustauschchromatographie und isoelektrische Fokussierung umfassen.

Aus den Chemical Abstracts 97, 319p, Seite 32 (1982) ist die Herstellung von Antithrombin III durch Spaltung des Antithrombin III-Heparin-Komplexes mit Protamin bekannt. Nach diesem Verfahren bildet sich ein Protamin-Heparin-Komplex und das Antithrombin wird unter Beibehaltung seiner ursprünglichen biologischen Aktivität freigesetzt.

Weiters ist von M. Miller-Andersson et al. in Throm. Res. 5, 439 (1974) eine Methode zur Gewinnung von Antithrombin III durch Affinitätschromatographie beschrieben, wobei Antithrombin III an Heparin-Agarose adsorbiert wird und eine weitere Reinigung über Ionenaustauschchromatographie und Gelfiltration erfolgt.

Aus T. Kitani et al., Thromb. Res. 17, 367-374 (1980) sind des weiteren analytische Untersuchungen zur Wechselwirkung von Protamin, Antithrombin III und Heparin bekannt geworden, dabei wurde heparinisiertes Plasma mit bzw. ohne Protaminzusatz einer Gelfiltration unterzogen und die Fraktionen auf ihren Gehalt an Protein, radioaktiv markiertem Heparin und Antithrombin III bzw. an Antithrombin III-Heparin-Komplex geprüft. Eine Isolierung bzw. Reindarstellung der einzelnen Bestandteile wurde nicht vorgenommen.

Die Erfindung stellt sich die Aufgabe, nach einem Verfahren der eingangs erwähnten Art Antithrombin III-Konzentrate mit hoher Reinheit und Ausbeute herstellen zu können, und ist dadurch gekennzeichnet, daß in der ersten Stufe der gebildete Antithrombin III-Heparin- bzw. Antithrombin III-Heparinoid-Komplex an einem Anionenaustauscher adsorbiert, das Adsorbat durch eine Salzlösung eluiert, der in dem Eluat enthaltene Antithrombin III-Heparin- bzw. Antithrombin III-Heparinoid-Komplex von Salzen und unerwünschten Proteinen getrennt wird und im zweiten Schritt die an dem gereinigten Komplex angereicherte Lösung des Antithrombin III-Heparin- bzw. Antithrombin III-Heparinoid-Komplexes mit immobilisiertem Protamin behandelt wird, wobei der Komplex gespalten und Heparin an dem immobiliserten Protamin gebunden wird, worauf der das Antithrombin III enthaltende Überstand durch Lyophilisieren in haltbare Form gebracht wird.

Die beschriebene Gewinnung des Antithrombin III aus dem Komplex, wobei Heparin gebunden bleibt, ist den bisher bekannten Verfahren zur Antithrombin III-Isolierung überlegen, da der Komplex einfach und in reiner Form erhalten werden kann. Eine Leakage des Liganden spielt keine Rolle, da Protamin klinisch zur Heparin-Neutralisierung angewendet wird und somit unbedenklich ist.

Das erfindungsgemäße Verfahren wird durch folgende Beispiele näher erläutert:

Beispiel:

Herstellung eines Antithrombin III-Heparin-Komplexes aus Plasma

Zu 100 l Plasma wurden nach der Entfernung des Kryopräzipitates 8.10⁵ E Heparin zugesetzt und es wurde 30 min bei +4°C gerührt. Nach dem Einrühren von 100 g DEAE-Sephadex A 50 wurde weitere 2 Stunden bei +4°C gerührt. Das beladene Gel wurde mittels Büchnertrichter abgetrennt und zur Entfernung von Begleitproteinen mit 2 l einer Phosphat-Citrat-gepufferten, isotonen Kochsalzlösung eines pH-Wertes von 7,5 gewaschen. Das beladene, gewaschene Gel wurde in 2,2 l des oben genannten Puffers suspendiert, und durch Zugabe von festem NaCl wurde eine Leitfähigkeit von 42 mS/cm eingestellt. Nach einstündigem Rühren bei +4°C wurde mittels Büchnertrichter abgetrennt und mit 0,7 l einer Phosphat- und Citrat-gepufferten Kochsalzlösung mit einer Leitfähigkeit von 42 mS/cm nachgewaschen.

Aus den vereinigten Eluaten wurde durch Zugabe von 1,4 kg Ammoniumsulfat und Einstellen des pH-Wertes auf 5,5 der Antithrombin III-Heparin-Komplex ausgefällt. Die hierbei angewendete Ammoniumsulfatkonzentration von 430 g/l entspricht einer 80 %igen Sättigung der Lösung. Nach einstündigem Rühren bei +4°C wurde der Niederschlag durch Filtration abgetrennt und gemeinsam mit 13,5 g NaCl und 221 g Na₃Citrat . 2 H₂O in 1,5 l destilliertem Wasser gelöst.

Der pH-Wert wurde auf 7,5 eingestellt und zur Inaktivierung von eventuell vorhandenen Viren wurde 10 Stunden auf 60°C erhitzt.

Das pasteurisierte Produkt wurde gegen 50 l isotone Kochsalzlösung dialysiert und zur weiteren Reinigung wurden bei einem pH-Wert von 7,0 303 g Ammoniumsulfat je 1 l eingerührt. Die hierbei angewendete Ammoniumsulfatkonzentration von 270 g/l entspricht einer 50 %igen Sättigung der Lösung. Nach 45 min Rührdauer bei +4°C wurde der Niederschlag durch Zentrifugieren abgetrennt und verworfen.

Der Überstand wurde gegen 100 l einer Citrat-gepufferten, isotonen Kochsalzlösung dialysiert und durch Ultrafiltration auf einen Antithrombin III-Gehalt von 50 E/ml eingeengt.

Nach der Dialyse gegen eine Citrat-gepufferte isotone Kochsalzlösung wurde sterilfiltriert, in Flaschen abgefüllt und gefriergetrocknet.

Die enzymatischen Aktivitäten von Antithrombin III und Heparin wurden in folgender Weise getestet:

Enzymatische Aktivität von Antithrombin III: Die Aktivität wurde auf eine Standardpräparation bezogen, die gegen Antithrombin III Plasma Human (1. Int. R.P. 72/1) eingeeicht worden war. Mit dieser Standardpräparation wurde eine Eichkurve ermittelt, auf der die zu testenden Proben abgelesen wurden.

Die Verdünnung der Proben und des Standards auf Aktivitäten zwischen 0,0125 und 0,0625 E/ml erfolgte mit einer Pufferlösung folgender Zusammensetzung:

```
     3,03 g  Tris       )
    10,8  g  NaCl        )
     1,4  g  Na-EDTA     )  je 1 l, pH 8,4
  3000     E Heparin     )
```

Pipettierschema:

    a) 0,1 ml Probe wurde auf 37°C erwärmt
    b) 0,1 ml Thrombin (12 IE/ml) wurden zugesetzt und es wurde 3 min bei 37°C inkubiert
    c) 0,1 ml einer 1,2 mM Lösung von TH-1 (2 AcOH.H-D-CHG-Ala-Arg-pNA) wurden zugesetzt
    d) nach genau 1 min bei 37°C wurde die Reaktion durch Zugabe von 0,1 ml 20 % Essigsäure gestoppt
    e) die Extinktion bei 405 nm wurde gemessen

Enzymatische Aktivität von Heparin:

Die Aktivität wurde auf eine Standardpräparation bezogen, die gegen den 3. Int. Stand. Heparin 65/69 eingeeicht worden war. Mit dieser Standardpräparation wurde eine Eichkurve ermittelt, auf der die zu testenden Proben abgelesen wurden.

Die Verdünnung der Proben und des Standards auf Aktivitäten von 0,001 bis 0,5 IE/ml erfolgte mit einer Pufferlösung folgender Zusammensetzung:

```
     6,06 g  Tris       )
    18,12 g  NaCl        )  je 1 l, pH 8,3
     2,79 g  Na-EDTA     )
```

Pipettierschema:

    a) 0,2 ml Probe und 0,2 ml heparinfreies Antithrombin III (3 E/ml) wurden 20 min bei Raumtemperatur und 3 bis 5 min bei 37°C inkubiert
    b) 0,2 ml Thrombin (20 IE/ml) wurden zugesetzt und es wurde 1,5 min bei 37°C inkubiert

c) 0,2 ml einer 1,2 mM Lösung von TH-1 wurden zugesetzt

d) nach genau 1 min bei 37°C wurde die Reaktion durch Zugabe von 0,2 ml 50 % Eisessig gestoppt

e) die Extinktion bei 405 nm wurde gemessen

Dabei ergab sich für das nach dem Beispiel 1 erhaltene Endprodukt ein Heparingehalt von 5 bis 7 Heparineinheiten pro Einheit Antithrombin III.

Herstellung von Antithrombin III aus dem Antithrombin III-Heparin-Komplex:

Für diese Arbeitsweise wird ein immobilisiertes Protamin, ein sogenanntes Protamin-Gel, benötigt. Solche Gele können in verschiedener Weise hergestellt werden. Im folgenden sind zwei Methoden angeführt.

Methode 1: Protamin-Sepharose

1 l eines quervernetzten 6 %igen Agarosegels (Sepharose Cl-6B) wird in 2 l einer 1 M $Na_2CO_3$-Lösung suspendiert und mit einer Lösung von 100 g BrCN in 100 ml Acetonitril bei einem pH-Wert von 11,0 bis 11,5 und einer Temperatur von 5 bis 10°C aktiviert.

Anschließend wird viermal mit je 3 l einer 0,2 M $NaHCO_3$-Lösung und zuletzt mit 3 l einer Borat-gepufferten Kochsalzlösung vom pH-Wert 9,5 gewaschen. Das BrCN-aktivierte Gel wird über Nacht mit 1 l der oben genannten Borat-Kochsalzlösung, der 20 g Protaminsulfat zugesetzt worden waren, bei einem pH-Wert von 9,5 und einer Temperatur von +4°C gerührt.

Nach der Blockierung der restlichen aktiven Gruppen mit 1 l einer 1 M Äthanolamin-Lösung (2 h bei Raumtemperatur) wird je dreimal abwechselnd mit je 3 l einer $NaHCO_3$-gepufferten Kochsalzlösung vom pH-Wert 8,5 und einer Acetat-gepufferten Kochsalzlösung vom pH-Wert 4,0 gewaschen.

Vor der Verwendung zur Entfernung von Heparin wird das Protamin-Gel mit einer Tris- und Citrat-gepufferten Kochsalzlösung vom pH-Wert 8,0 äquilibriert.

Methode 2: Protamin-Eupergit

5 g Oxiran-Acrylharz (Eupergit C) werden mit einer Lösung von 400 mg Protaminsulfat in 20 ml 1 M Kaliumphosphat vom pH-Wert 9,5 versetzt und 16 h bei Raumtemperatur stehen gelassen. Nach Waschen mit Wasser, 1 M NaCl-Lösung und 0,01 M Phosphatlösung werden die restlichen reaktiven Gruppen mit 70 ml einer 5 %igen Lösung von Beta-Mercaptoäthanol blockiert (16 h bei Raumtemperatur). Schließlich wird fünfmal mit je 80 ml Wasser gewaschen. Vor der Verwendung zur Entfernung von Heparin wird wie bei Methode 1 äquilibriert.

Der wie oben beschrieben hergestellte Antithrombin III-Heparin-Komplex wurde gelöst bzw. wurde die Lösung verwendet, die nach der Dialysierung erhalten wurde. Zu je 1 l dieser dialysierten Lösung wurde 1 g Tris zugesetzt, der pH-Wert wurde auf 8,0 eingestellt und es wurden 15 ml des nach Methode 1 hergestellten Protamin-Gels zugesetzt. Nach dem Rühren über Nacht wurde das Gel durch Filtration abgetrennt; die Hauptmenge des heparinfreien Antithrombin III befand sich im Adsorptionsüberstand.

Zur Erhöhung der Ausbeute an Antithrombin III wurde das Gel mit Tris- und Citrat-gepufferten Kochsalzlösungen von steigender Ionenstärke gewaschen. Der Adsorptionsüberstand und die heparinfreien Waschüberstände wurden zur Isolierung von Antithrombin III vereinigt und zur Konzentration lyophilisiert. Das so erhaltene Pulver wurde in 1 ml destilliertem Wasser je 1 g suspendiert und gegen eine Citrat-gepufferte, isotone Kochsalzlösung dialysiert. Nach der Dialyse wurde auf einen Antithrombin III-Gehalt von 50 E/ml verdünnt, es wurde sterilfiltriert, in Flaschen abgefüllt und gefriergetrocknet.

Die enzymatische Analyse lieferte einen Heparingehalt von kleiner 0,5 E Heparin je 1 E Antithrombin III.

**Patentansprüche**

1. Verfahren zur Herstellung eines Antithrombin III-Konzentrats, wobei zuerst Humanplasma oder Antithrombin III-haltige Plasmafraktionen unter Bildung eines Antithrombin III-Heparin- bzw. Antithrombin III-Heparinoid-Komplexes mit Heparin bzw. Heparinoid versetzt wird und anschließender Spaltung des Antithrombin III-Heparin- bzw. Antithrombin III-Heparinoid-Komplexes mit Protamin, dadurch gekennzeichnet, daß in der ersten Stufe der gebildete Antithrombin III-Heparin- bzw. Antithrombin III-Heparinoid-Komplex an einem Anionenaustauscher adsorbiert, das Adsorbat durch eine Salzlösung eluiert, der in dem Eluat enthaltene Antithrombin III-Heparin- bzw. Antithrombin III-Heparinoid-Komplex von Salzen und unerwünschten Proteinen getrennt wird und im zweiten Schritt die an dem gereinigten Komplex angereicherte Lösung des Antithrombin III-Heparin- bzw. Antithrombin III-Heparinoid-Komplexes mit immobilisiertem Protamin behandelt wird, wobei der Komplex gespalten und Heparin an dem immobiliserten Protamin gebunden wird, worauf der das Antithrombin III enthaltende Überstand durch Lyophilisieren in haltbare Form gebracht wird.

## Claims

1. Method of producing an antithrombin III concentrate, wherein human plasma or antithrombin III-containing plasma fractions are at first admixed with heparin or heparinoid under formation of an antithrombin III-heparin complex or antithrombin III-heparinoid complex, respectively, and subsequent cleavage of the antithrombin III-heparin complex or antithrombin III-heparinoid complex with protamine, characterized in that, in the first stage, the antithrombin III-heparin or antithrombin III-heparinoid complex formed is adsorbed on an anion exchanger, the adsorbate is eluted by means of a salt solution, the antithrombin III-heparin or antithrombin III-heparinoid complex contained in the eluate is separated from salts and undesired proteins, and, in the second stage, the solution of the antithrombin III-heparin or antithrombin III-heparinoid complex, enriched with the purified complex is treated with immobilized protamine, thus cleaving the complex and binding heparin to the immobilized protamine, whereupon the antithrombin III-containing supernatant is brought into a stable form by lyophilization.

## Revendications

1. Procédé de préparation d'un concentré d'antithrombine III, dans lequel tout d'abord du plasma humain ou des fractions de plasma contenant de l'antithrombine III sont additionnés d'héparine ou d'héparinoïde avec formation d'un complexe antithrombine III-héparine ou antithrombine III-héparinoïde puis le complexe antithrombine III-héparine ou antithrombine III-héparinoïde est coupé par la protamine, caractérisé en ce que, dans la première étape, le complexe antithrombine III-héparine ou antithrombine III-héparinoïde formé est adsorbé sur un échangeur d'anions, l'adsorbat est élué par une solution saline, le complexe antithrombine III-héparine ou antithrombine III-héparinoïde contenu dans l'éluat est débarrassé des sels et des protéines indésirables, et, dans la deuxième étape, la solution de complexe antithrombine III-héparine ou antithrombine III-héparinoïde enrichie en complexe purifié est traitée par de la protamine immobilisée, de sorte que le complexe est coupé et l'héparine est liée à la protamine immobilisée, après quoi le surnageant contenant l'antithrombine III est mis sous forme conservable par lyophilisation.